# FASCICULE DE BREVET EUROPEEN

(11) **EP 1 240 162 B1**
(45) Date de publication et mention de la délivrance du brevet: **14.05.2003**
(21) Numéro de dépôt: 00990091.1
(22) Date de dépôt: 21.12.2000
(51) Int. Cl.: C07D 409/14

(54) **NOUVEAUX DERIVES DE 3-AMINO-1,2-DITHIOLES, LEUR PROCEDE DE PREPARATION ET LES COMPOSITIONS PHARMACEUTIQUES QUI LES CONTIENNENT**
3-IMINO-1,2-DITHIOLEN DERIVATE, VERFAHREN ZU DEREN HERSTELLUNG UND DIESE ENTHALTENDE PHARMAZEUTISCHE ZUSAMMENSTELLUNGEN
NOVEL DERIVATIVES OF 3-IMINO-1,2-DITHIOLES, METHOD FOR PRODUCING THEM AND PHARMACEUTICAL COMPOSITIONS CONTAINING THE SAME

(30) Priorité: 22.12.1999 FR 9916194
(43) Date de publication de la demande: 18.09.2002
(73) Titulaire: Les Laboratoires Servier, 92415 Courbevoie Cedex (FR)
(72) Inventeur: DUFLOS, Muriel, F- 44640 Vue (FR); ROBERT, Jean-Michel, F-44000 Nantes (FR); NOURRISSON, Marie-Renée, F-44240 La Chapelle sur Erdre (FR); LE BAUT, Guillaume, F-44230 Saint Sebastien sur Loire (FR); CAIGNARD, Daniel-Henri, F-78230 Le Pecq (FR); BIZOT-ESPIARD, Jean-Guy, F-75015 Paris (FR); RENARD, Pierre, F-78150 Le Chesnay (FR)
(86) Numéro de dépôt international: FR0003632
(87) Numéro de publication internationale: WO01046180

(56) Documents cités:
- J.A. MITCHELL: "STUDIES OF HETEROCYCLIC COMPOUNDS.PART 28." JOURNAL OF THE CHEMICAL SOCIETY, PERKIN TRANSACTIONS 1., vol. 2, 1982, pages 499-507, XP002148231 CHEMICAL SOCIETY. LETCHWORTH., GB ISSN: 1470-4358

## Description

La présente invention concerne de nouveaux dérivés de 3-imino-1,2-dithioles, leur procédé de préparation, les compositions pharmaceutiques qui les contiennent ainsi que leur utilisation en tant qu'antiinflammatoires, et plus particulièrement en tant qu'antipsoriatiques.

A ce jour, la demanderesse ne connaît aucun composé antiinflammatoire de structure proche de celle des produits de l'invention.

Ceux-ci, outre le fait qu'ils soient particulièrement originaux, présentent des propriétés antiinflammatoires qui se manifestent non seulement après administration par voie systémique, mais également par voie topique, ce qui les rend particulièrement intéressants dans des maladies cutanées telles que, par exemple, le psoriasis, l'acné ou les dermatites.

Le psoriasis est une maladie de la peau caractérisée par une hyperprolifération des kératinocytes de l'épiderme. Elle évolue sur un mode chronique entrecoupé de rémissions et est associée à une inflammation.

A l'heure actuelle, les thérapies appliquées contre cette maladie (rétinoïdes, arotinoïdes, glucocorticoïdes, analogues de la vitamine D, irradiations UVA) ne sont que partiellement efficaces et exercent des effets néfastes.

Il était donc particulièrement intéressant de synthétiser de nouveaux composés à la fois plus actifs et dénués de toxicité.

Plus spécifiquement, la présente invention concerne les composés de formule (I): 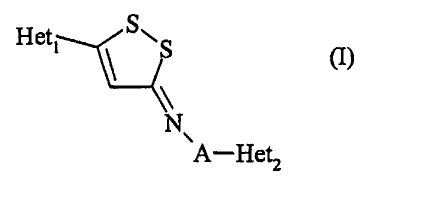 dans laquelle :
- Het₁ et Het₂, identiques ou différents, représentent chacun un groupement hétéroaryle,
- A représente une liaison ou un groupement alkylène (C₁-C₆) linéaire ou ramifié,
leurs isomères optiques lorsqu'ils existent ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

Par groupement hétéroaryle, on entend un groupement aromatique mono- ou bicyclique de 5 à 12 chaînons contenant un, deux ou trois hétéroatomes choisis parmi oxygène, azote ou soufre étant entendu que l'hétéroaryle peut être éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, nitro ou amino (substitué éventuellement par un ou plusieurs groupements alkyle (C₁-C₆) linéaire ou ramifié). Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, quinolyle, isoquinolyle, pyrimidinyle, benzimidazolyle.

Le groupement Het₁ préféré de la formule (I) est le groupement pyridyle éventuellement substitué.

Le groupement Het₂ préféré de la formule (I) est le groupement pyridyle éventuellement substitué.

Le composé préféré de formule (I) est le 3-(3-pyridyl)-5-[N-(4,6-diméthyl-2-pyridyl)-imino]-1,2-dithiole.

L'invention s'étend également au procédé de préparation des composés de formule (I) caractérisé en ce que l'on met en réaction un composé de formule (II) : 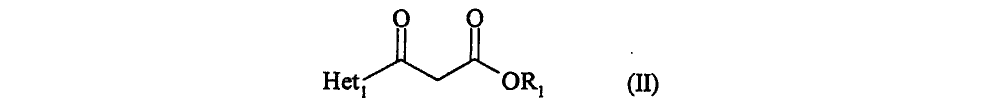 dans laquelle Het₁ a la même signification que dans la formule (I), et R₁ représente un groupement alkyle (C₁-C₆) linéaire ou ramifié,
avec un composé de formule (III):

H₂N-A-Het₂ (III)

dans laquelle Het₂ et A ont la même signification que dans la formule (I),
pour conduire au composé de formule (IV) : 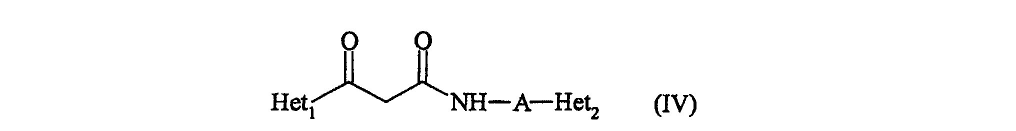 dans laquelle Het₁, Het₂ et A ont la même signification que dans la formule (I),
que l'on met en réaction avec un agent de thionation pour conduire au composé de formule (I),
que l'on purifie, le cas échéant, selon une technique classique de purification, et que l'on transforme, si on le souhaite, en ses sels d'addition à un acide pharmaceutiquement acceptable.

Le composé de formule (II) est obtenu à partir du composé de formule (V) : 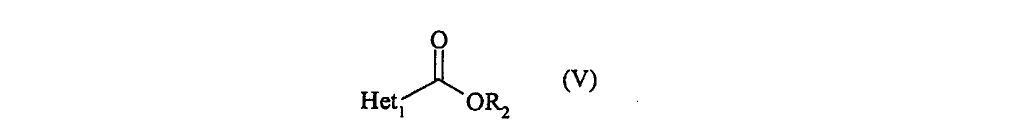 dans laquelle Het₁ a la même signification que dans la formule (I), et R₂ représente un groupement alkyle (C₁-C₆) linéaire ou ramifié,
selon le procédé décrit dans J. Org. Chem. 1983, 48, 5007.

L'invention s'étend également à un procédé particulier de préparation du 3-(3-pyridyl)-5-[N-(4,6-diméthyl-2-pyridyl)-imino]-1,2-dithiole, caractérisé en ce que l'on met en réaction un composé de formule (IIa), cas particulier des composés de formule (II): 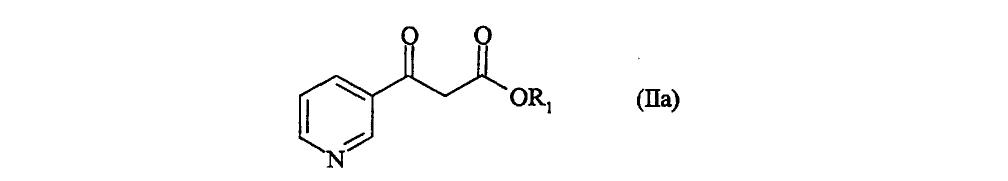 dans laquelle R₁ a la même signification que précédemment,
avec le composé de formule (IIIa), cas particulier des composés de formule (III) : 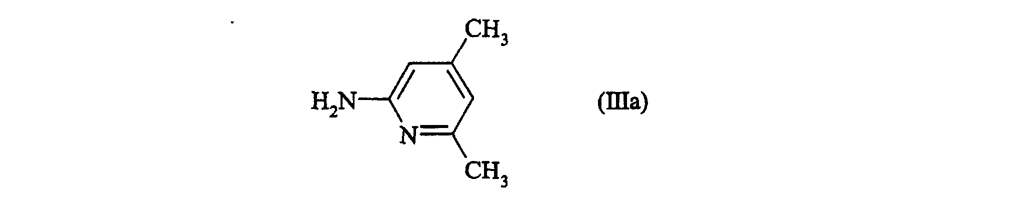 pour conduire au composé de formule (IVa), cas particulier des composés de formule (IV) : 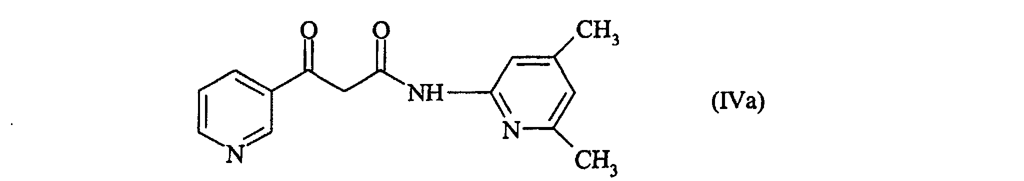 que l'on met en réaction avec un agent de thionation tel que, par exemple, le réactif de Lawesson, pour conduire au 3-(3-pyridyl)-5-[N-(4,6-diméthyl-2-pyridyl)-imino]-1,2-dithiole,
que l'on purifie selon une technique classique de purification, et que l'on transforme si on le souhaite en ses sels d'addition à un acide pharmaceutiquement acceptable.

Le N-(4,6-diméthyl-2-pyridyl)-3-oxo-3-(3-pyridyl)-propionamide, composé de formule (IVa), est nouveau et fait également partie de l'invention à titre d'intermédiaire de synthèse du 3-(3-pyridyl)-5-[N-(4,6-diméthyl-2-pyridyl)-imino]-1,2-dithiole.

Les composés de formule (I), outre le fait qu'ils soient nouveaux, présentent des propriétés pharmacologiques intéressantes.

L'étude de ces propriétés a en effet montré que les dérivés de formule (I) n'étaient pas toxiques, et étaient doués d'activité antiinflammatoire, qui se manifeste aussi bien par voie topique que systémique.

Ce spectre d'activité rend donc les composés de la présente invention utiles dans le traitement de l'arthrite chronique ou aiguë et dans un certain nombre d'indications telles que les rhumatismes inflammatoires, la polyarthrite rhumatoïde, la spondylarthrite ankylosante, les arthroses, les rhumatismes articulaires, les lombalgies. De par leur activité par voie topique, les composés de l'invention sont utiles dans le traitement de certains troubles cutanés tels que, par exemple, le psoriasis, l'acné et les dermatites.

La présente invention a également pour objet les compositions pharmaceutiques contenant un composé de formule (I), ou un de ses sels d'addition à un acide pharmaceutiquement acceptable, en combinaison avec un ou plusieurs excipients inertes, non toxiques et appropriés.

Parmi les compositions pharmaceutiques selon l'invention, on pourra citer plus particulièrement, à titre d'exemples et de façon non limitative, celles qui conviennent à l'administration orale, parentérale, nasale, rectale, perlinguale, oculaire, cutanée, transcutanée, percutanée ou pulmonaire et notamment les préparations injectables, les aérosols, les gouttes oculaires ou nasales, les suppositoires, les comprimés simples, pelliculés ou dragéifiés, les gélules, les capsules, les crèmes, pommades, et gels dermiques.

La posologie utile varie selon l'âge, le sexe et le poids du patient, la voie d'administration, la nature de l'affection et des traitements éventuellement associés et s'échelonne entre 1 mg et 5 grammes par 24 heures, de préférence entre 1 mg et 500 mg par 24 h.

Les exemples suivants illustrent l'invention et ne la limitent en aucune façon.

Les produits de départ utilisés sont des produits connus ou préparés selon des modes préparatoires connus.

Les préparations A à D conduisent à des intermédiaires de synthèse, utiles pour la préparation des composés de l'invention.

Les structures des composés décrits dans les exemples ont été déterminées selon les techniques spectrométriques usuelles (infrarouge, RMN, spectrométrie de masse).

### PREPARATION A : 3-Oxo-3-(4-pyridyl)-propionate de méthyle

Le produit attendu est obtenu selon le procédé décrit dans J. Org. Chem. 1983, 48, 5007 à partir d'isonicotinate de méthyle.

### PREPARATION B : 3-Oxo-3-(2-pyrazinyl)-propionate de méthyle

Le produit attendu est obtenu selon le procédé décrit dans J. Org. Chem. 1983, 48, 5007 à partir de pyrazine-2-carboxylate de méthyle.

### PREPARATION C : 3-Oxo-3-(5-méthyl-2-thiényl)-propionate de méthyle

Le produit attendu est obtenu selon le procédé décrit dans J. Org. Chem. 1983, 48, 5007 à partir de 5-méthyl-thiophène-2-carboxylate de méthyle.

### PREPARATION D : 3-Oxo-3-(2-furyl)-propionate de méthyle

Le produit attendu est obtenu selon le procédé décrit dans J. Org. Chem. 1983, 48, 5007 à partir de furane-2-carboxylate de méthyle.

### EXEMPLE 1 : 3-(3-Pyridyl)-5-[N-(4,6-diméthyl-2-pyridyl)-imino]-1,2-dlthiole

### Stade A : N-(4,6-Diméthyl-2-pyridyl)-3-oxo-3-(3-pyridyl)-propionamide

Le produit attendu est obtenu selon le procédé décrit dans J. Chem. Soc. (C) 1969, 89 à partir de 2-amino-4,6-diméthyl-pyridine et de 3-oxo-3-(3-pyridyl)-propionate de méthyle décrit dans J. Org. Chem. 1983, 48, 5007.

### Stade B : 3-(3-Pyridyl)-5-[N-(4,6-diméthyl-2-pyridyl)-imino]-1,2-dithiole

A 10 mmoles du composé obtenu dans le stade précédent en solution dans le toluène sont ajoutées à 120°C 12 mmoles de réactif de Lawesson. Après 45 minutes de chauffage, le toluène est évaporé et le résidu brut obtenu est purifié, d'abord par chromatographie sur silice en utilisant comme éluant de l'éther éthylique puis un mélange chlorure de méthylène/éthanol 98/2, puis par cristallisation dans l'éther isopropylique, pour conduire au produit attendu.
Point de fusion : 152°C

### EXEMPLE 2 : 3-[3-Pyridyl]-5-[N-(5-méthyl-1,3-thiazol-2-yl)-imino]-1,2-dithiole

Le produit attendu est obtenu selon le procédé décrit dans l'Exemple 1 à partir de 2-amino-5-méthyl-1,3-thiazole et de 3-oxo-3-(3-pyridyl)-propionate de méthyle décrit dans J. Org. Chem. 1983, 48, 5007.

### EXEMPLE 3 : 3-(3-Pyridyl)-5-[N-(pyrimidin-2-yl)-imino]-1,2-dithiole

Le produit attendu est obtenu selon le procédé décrit dans l'Exemple 1 à partir de 2-amino-pyrimidine et de 3-oxo-3-(3-pyridyl)-propionate de méthyle décrit dans J. Org. Chem. 1983, 48, 5007.

### EXEMPLE 4 : 3-(4-Pyridyl)-5-[N-(5-chloro-2-pyridyl)-imino]-1,2-dithiole

Le produit attendu est obtenu selon le procédé décrit dans l'Exemple 1 à partir du composé décrit dans la Préparation A et de 2-amino-5-chloropyridine.

### EXEMPLE 5 : 3-(2-Pyrazinyl)-5-[N-(4-méthoxy-6-méthyl-pyrimidin-2-yl)-imino]-1,2-dithiole

Le produit attendu est obtenu selon le procédé décrit dans l'Exemple 1 à partir du composé décrit dans la Préparation B et de 2-amino-4-méthoxy-6-méthyl-pyrimidine.

### EXEMPLE 6 : 3-(5-Méthyl-2-thiényl)-5-[N-(5-nitro-1,3-thiazol-2-yl)-imino]-1,2-dithiole

Le produit attendu est obtenu selon le procédé décrit dans l'Exemple 1 à partir du composé décrit dans la Préparation C et de 2-amino-5-nitro-1,3-thiazole.

### EXEMPLE 7 : 3-(2-Furyl)-5-[N-(2-pyrazinyl)-imino]-1,2-dithiole

Le produit attendu est obtenu selon le procédé décrit dans l'Exemple 1 à partir du composé décrit dans la Préparation D et de 2-aminopyrazine.

### EXEMPLE 8 : 3-(3-Pyridyl)-5-[N-((3-pyridyl)-méthyl)-imino]-1,2-dithiole

Le produit attendu est obtenu selon le procédé décrit dans l'Exemple 1 à partir de 3-oxo-(3-pyridyl)-propionate de méthyle décrit dans J. Org. Chem. 1983, 48, 5007 et de 3-picolylamine.

### ETUDE PHARMACOLOGIOUE DES COMPOSES DE L'INVENTION

### EXEMPLE 9 : Mise en évidence de l'activité anti-inflammatoire cutanée (topique) en aigu

L'ester de phorbol (Phorbol 12-myristate 13-acetate) (5 µg) est appliqué en topique sur les surfaces antérieures et postérieures de l'oreille droite de la souris 30 minutes après application du véhicule (éthanol 95 %) ou de l'agent. La différence d'épaisseur entre l'oreille droite et l'oreille gauche (oedème) est mesurée 3 heures 30 après l'application de l'ester de phorbol.

Le pourcentage d'inhibition de l'inflammation cutanée par rapport à un groupe d'animaux traité à l'éthanol 95 % en topique est calculé. Les composés de l'invention permettent une diminution significative de l'inflammation dès la concentration de 30 µg/oreille.

### EXEMPLE 10 : Mise en évidence de l'activité anti-inflammatoire cutanée (topique) en chronique curatif (modèle de psoriasis)

L'ester de phorbol (1 µg) est appliqué en topique sur la totalité de la surface externe de l'oreille droite de la souris aux jours 0, 2, 4, 7 et 9. Les composés à étudier ou le véhicule sont appliqués en topique 2 fois par jour à la dose de 2000 µg/10 µl à chaque application, les jours 7, 8 et 9 et une seule fois le 10^{ème} jour.

La différence d'épaisseur entre les oreilles droite et gauche, qui permet d'apprécier l'importance des lésions cutanées et de l'inflammation, est mesurée à J₀, J₂, J₄, J₇ et J₉, 6 heures après l'application d'ester de phorbol.

Dès le premier jour d'application, les composés de l'invention permettent une diminution significative de l'inflammation de plus de 40% (p < 0,001). L'effet se poursuit et s'accentue jusqu'à la fin de l'étude pour atteindre à J₁₀ une diminution de l'inflammation supérieure à 55 % (p<0,001).

### COMPOSITIONS PHARMACEUTIQUES

### EXEMPLE 11 : Comprimés pour le traitement des maladies inflammatoires

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

### EXEMPLE 12 : Pommade destinée au traitement du psoriasis

| Formule de préparation pour 100 kg de pommade dosée à 1 % | |
|---|---|
| Composé de l'exemple 1 | 1000 g |
| Excipient en quantité suffisante pour 100 kg : | |
| Alcools cétylique, stéarylique, isopropylique ; | |
| lanoline, | |
| monostéarate de poyléthylène glycol, | |
| eau distillée de laurier cerise | |

## Revendications

1. Composé de formule (I) : 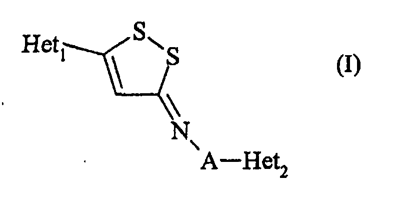 dans laquelle :
- Het₁ et Het₂, identiques ou différents, représentent chacun un groupement hétéroaryle,
- A représente une liaison ou un groupement alkylène (C₁-C₆) linéaire ou ramifié,
ses isomères optiques lorsqu'ils existent ainsi que ses sels d'addition à un acide pharmaceutiquement acceptable,
étant entendu que par groupement hétéroaryle, on entend un groupement aromatique mono- ou bicyclique de 5 à 12 chaînons contenant un, deux ou trois hétéroatomes choisis parmi oxygène, azote ou soufre étant entendu que l'hétéroaryle peut être éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, nitro ou amino (substitué éventuellement par un ou plusieurs groupements alkyle (C₁-C₆) linéaire ou ramifié).

2. Composé de formule (I) selon la revendication 1 tel que Het₁ représente un groupement pyridyle éventuellement substitué, ses isomères optiques lorsqu'ils existent ainsi que ses sels d'addition à un acide pharmaceutiquement acceptable.

3. Composé de formule (I) selon la revendication 1 tel que Het₂ représente un groupement pyridyle éventuellement substitué, ses isomères optiques lorsqu'ils existent ainsi que ses sels d'addition à un acide pharmaceutiquement acceptable.

4. Composé de formule (I) selon la revendication 1 qui est le 3-(3-pyridyl)-5-[N-(4,6-diméthyl-2-pyridyl)-imino]-1,2-dithiole, ainsi que ses sels d'addition à un acide pharmaceutiquement acceptable.

5. Procédé de préparation des composés de formule (I) selon la revendication 1 **caractérisé en ce que** l'on met en réaction un composé de formule (II) : 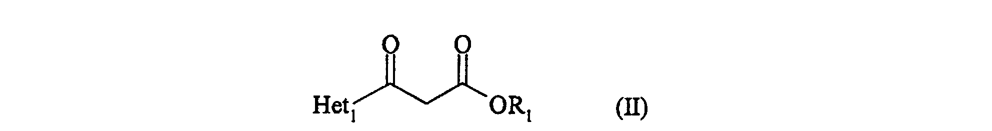 dans laquelle Het₁ a la même signification que dans la formule (I), et R₁ représente un groupement alkyle (C₁-C₆) linéaire ou ramifié,
avec un composé de formule (III) :
H₂N-A-Het₂ (III)
dans laquelle Het₂ et A ont la même signification que dans la formule (I),
pour conduire au composé de formule (IV) : 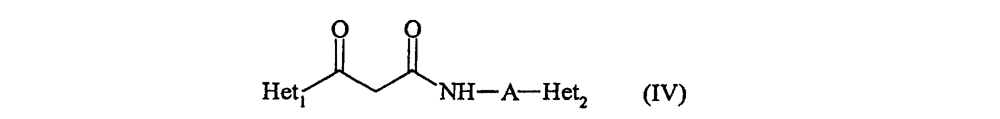 dans laquelle Het₁, Het₂ et A ont la même signification que dans la formule (I),
que l'on met en réaction avec un agent de thionation pour conduire au composé de formule (I),
que l'on purifie, le cas échéant, selon une technique classique de purification, et que l'on transforme, si on le souhaite, en ses sels d'addition à un acide pharmaceutiquement acceptable.

6. Procédé de préparation selon la revendication 5 du 3-(3-pyridyl)-5-[N-(4,6-diméthyl-2-pyridyl)-imino]-1,2-dithiole **caractérisé en ce que** l'on met en réaction un composé de formule (IIa), cas particulier des composés de formule (II) : 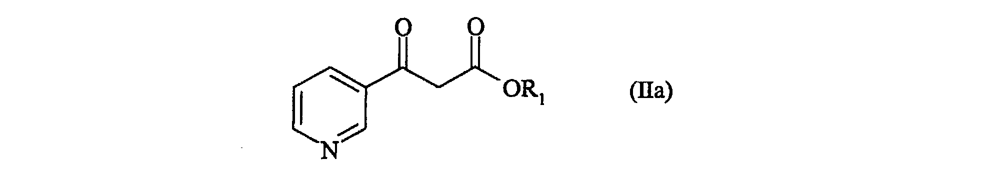 dans laquelle R₁ a la même signification que précédemment,
avec le composé de formule (IIIa), cas particulier des composés de formule (III) : 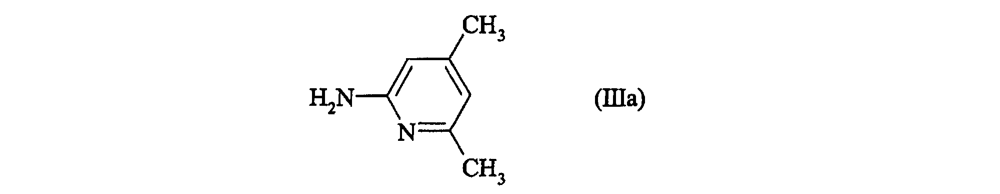 pour conduire au composé de formule (IVa), cas particulier des composés de formule (IV) : 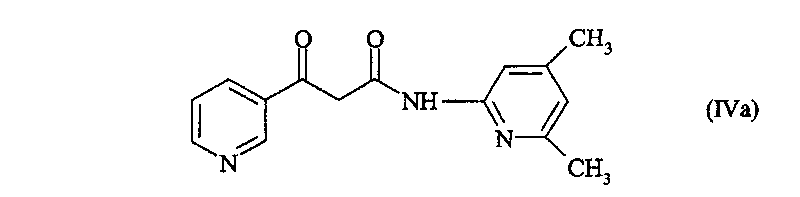 que l'on met en réaction avec un agent de thionation tel que, par exemple, le réactif de Lawesson, pour conduire au 3-(3-pyridyl)-5-[N-(4,6-diméthyl-2-pyridyl)-imino]-1,2-dithiole,
que l'on purifie selon une technique classique de purification, et que l'on transforme si on le souhaite en ses sels d'addition à un acide pharmaceutiquement acceptable.

7. Intermédiaire de synthèse du 3-(3-pyridyl)-5-[N-(4,6-diméthyl-2-pyridyl)-imino]-1,2-dithiole qui est le N-(4,6-diméthyl-2-pyridyl)-3-oxo-3-(3-pyridyl)-propionamide.

8. Compositions pharmaceutiques contenant comme principe actif un composé selon l'une quelconque des revendications 1 à 4, seul ou en combinaison avec un ou plusieurs véhicules inertes, non toxiques et pharmaceutiquement acceptables.

9. Compositions pharmaceutiques selon la revendication 8 utiles pour la fabrication de médicaments antiinflammatoires.

10. Compositions pharmaceutiques selon la revendication 9 utiles pour la fabrication de médicaments utiles dans le traitement du psoriasis.

## Patentansprüche

1. Verbindung der Formel (I): 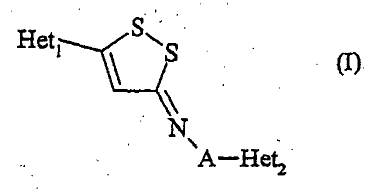 in der:
- Het₁ und Het₂, die gleichartig oder verschieden sind, jeweils eine Heteroarylgruppe und
- A eine Bindung oder eine geradkettige oder verzweigte (C₁-C₆)-Alkylengruppe bedeuten,
deren optische Isomere, falls sie existieren, sowie deren Additionssalze mit einer pharmazeutisch annehmbaren Säure,
mit der Maßgabe, daß unter einer Heteroarylgruppe eine mono- oder bicyclische aromatische Gruppe mit 5 bis 12 Kettengliedern, die ein, zwei oder drei Heteroatome ausgewählt aus Sauerstoff, Stickstoff oder Schwefel enthalten, zu verstehen ist, wobei es sich versteht, daß die Heteroarylgruppe gegebenenfalls substituiert sein kann durch ein oder mehrere gleichartige oder verschiedene Atome oder Gruppen ausgewählt aus Halogenatomen, geradkettigen oder verzweigten (C₁-C₆)-Alkylgruppen, Hydroxygruppen, geradkettigen oder verzweigten (C₁-C₆)-Alkoxygrüppen, geradkettigen oder verzweigten (C₁-C₆)-Polyhalogenalkylgruppen, Nitrogruppen oder Aminogruppen (die gegebenenfalls durch eine oder mehrere geradkettige oder verzweigte (C₁-C₆)-Alkylgruppen substituiert sind).

2. Verbindung der Formel (I) nach Anspruch 1, worin Het₁ eine gegebenenfalls substituierte Pyridylgruppe bedeutet, deren optische Isomere, falls sie existieren, sowie deren Additionssalze mit einer pharmazeutisch annehmbaren Säure.

3. Verbindung der Formel (I) nach Anspruch 1, worin Het₂ eine gegebenenfalls substituierte Pyridylgruppe bedeutet, deren optische Isomere, falls diese existieren, sowie deren Additionssalze mit einer pharmazeutisch annehmbaren Säure.

4. Verbindung der Formel (I) nach Anspruch 1, nämlich 3-(3-Pyridyl)-5-[N-(4,6-dimethyl-2-pyridyl)-imino]-1,2-dithiol sowie dessen Additionssälze mit einer pharmazeutisch annehmbaren Säure.

5. Verfahren zur Herstellung der Verbindungen der Formel (I) nach Anspruch 1, **dadurch gekennzeichnet, daß** man eine Verbindung der Formel (II): 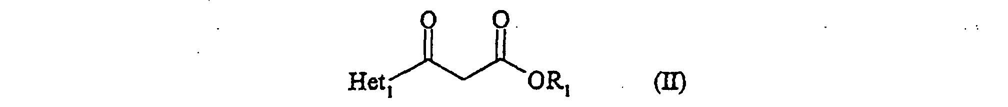 in der Het₁ die bezüglich der Formel (I) angegebenen Bedeutungen besitzt und R₁ eine geradkettige oder verzweigte (C₁-C₆)-Alkylgruppe bedeutet,
mit einer Verbindung der Formel (III):
H₂N - A - Het₂ (III)
in der Het₂ und A die bezüglich der Formel (I) angegebenen Bedeutungen besitzen, umsetzt zur Bildung der Verbindung der Formel (IV): 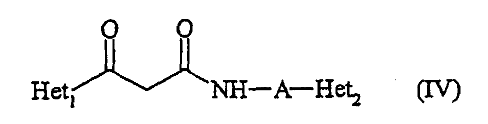 in der Het₁, Het₂ und A die bezüglich der Formel (I) angegebenen Bedeutungen besitzen,
welche man mit einem Thionierungsmittel umsetzt zur Bildung der Verbindung der Formel (I),
welche man gegebenenfalls mit Hilfe einer klassischen Reinigungsmethode reinigt und welche man gewünschtenfalls in ihre Additionssalze mit einer pharmazeutisch annehmbaren Säure umwandelt.

6. Verfahren zur Herstellung nach Anspruch 5 von 3-(3-Pyridyl)-5-[N-(4,6-dimethyl-2-pyridyl)-imino]-1,2-dithiol, **dadurch gekennzeichnet, daß** man eine Verbindung der Formel (IIa), einem Sonderfall der Verbindungen der Formel (II): 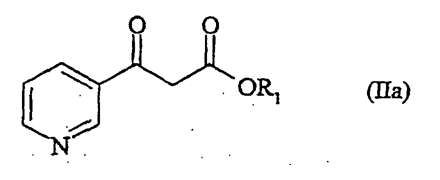 in der R₁ die oben angegebenen Bedeutungen besitzt,
mit der Verbindung der Formel (IIIa), einem Sonderfall der Verbindungen der Formel (III): 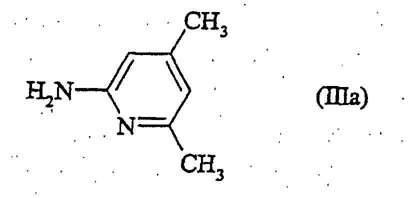 umsetzt zur Bildung der Verbindung der Formel (IVa), einem Sonderfall der Verbindungen der Formel (IV): 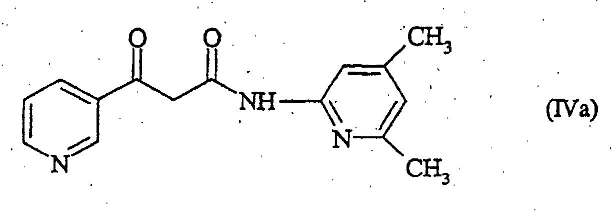 welche man mit einem Thionierungsmittel, wie beispielsweise dem Lawesson-Reagens, umsetzt zur Bildung von 3-(3-Pyridyl)-5-[N-(4,6-dimethyl-2-pyridyl)-imino]-1,2-dithiol,
welches man mit Hilfe einer klassischen Reinigungsmethode reinigt und gewünschtenfalls in eines seiner Additionssalze mit einer pharmazeutisch annehmbaren Säure umwandelt.

7. N-(4,6-Dimethyl-2-pyridyl)-3-oxo-3-(3-pyridyl)-propionamid als Zwischenprodukt für die Synthese von 3-(3-Pyridyl)-5-[N-(4,6-dimethyl-2-pyridyl)-imino]-1,2-dithiol.

8. Pharmazeutische Zubereitungen enthaltend als Wirkstoff eine Verbindung nach irgendeinem der Ansprüche 1 bis 4 allein oder in Kombination mit einem oder mehreren inerten, nichttoxischen und pharmazeutisch annehmbaren Trägermaterialien.

9. Pharmazeutische Zubereitungen nach Anspruch 8 nützlich für die Herstellung von antiinflammatorischen Arzneimitteln.

10. Pharmazeutische Zubereitungen nach Anspruch 9 nützlich für die Herstellung von Arzneimitteln für die Behandlung der Psoriasis.

## Claims

1. Compound of formula (I): 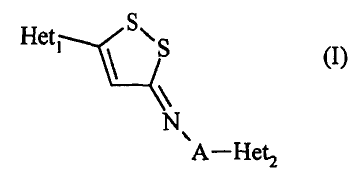 wherein:
- Het₁ and Het₂, which may be identical or different, each represents a heteroaryl group,
- A represents a bond or a linear or branched (C₁-C₆)alkylene group,
its optical isomers, where they exist, and its addition salts with a pharmaceutically acceptable acid,
wherein a heteroaryl group is to be understood as meaning an aromatic mono- or bicyclic group having from 5 to 12 ring members containing one, two or three hetero atoms selected from oxygen, nitrogen and sulphur, wherein the heteroaryl group may optionally be substituted by one or more identical or different atoms or groups selected from the halogen atoms and the groups linear or branched (C₁-C₆)alkyl, hydroxy, linear or branched (C₁-C₆)alkoxy, linear or branched (C₁-C₆)polyhaloalkyl, nitro and amino (optionally substituted by one or more linear or branched (C₁-C₆)alkyl groups).

2. Compound of formula (I) according to claim 1, wherein Het₁ represents an optionally substituted pyridyl group, its optical isomers, where they exist, and its addition salts with a pharmaceutically acceptable acid.

3. Compound of formula (I) according to claim 1, wherein Het₂ represents an optionally substituted pyridyl group, its optical isomers, where they exist, and its addition salts with a pharmaceutically acceptable acid.

4. Compound of formula (I) according to claim 1, which is 3-(3-pyridyl)-5-[N-(4,6-dimethyl-2-pyridyl)-imino]-1,2-dithiol, and addition salts thereof with a pharmaceutically acceptable acid.

5. Process for the preparation of compounds of formula (I) according to claim 1, **characterised in that** a compound of formula (II): 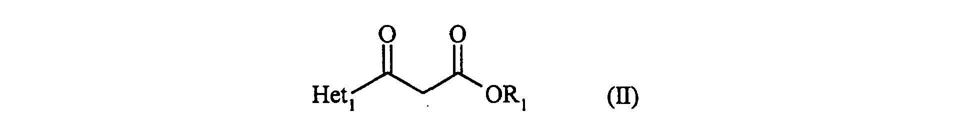 wherein Het₁ has the same meaning as in formula (I) and R₁ represents a linear or branched (C₁-C₆)alkyl group, is reacted
with a compound of formula (III):
H₂N-A-Het₂ (III)
wherein Het₂ and A have the same meanings as in formula (I),
to yield a compound of formula (IV) : 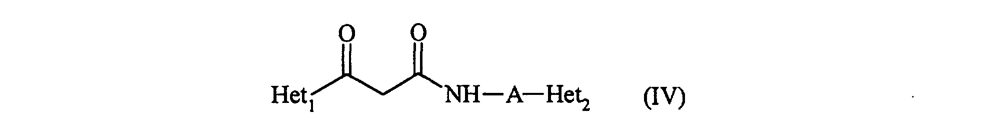 wherein Het₁, Het₂ and A have the same meanings as in formula (I),
which is reacted with a thionation agent to yield a compound of formula (I),
which is purified, if necessary, according to a conventional purification technique, and which is converted, if desired, into addition salts with a pharmaceutically acceptable acid.

6. Process according to claim 5 for the preparation of 3-(3-pyridyl)-5-[N-(4,6-dimethyl-2-pyridyl)-imino]-1,2-dithiol, **characterised in that** a compound of formula (IIa), a particular case of the compounds of formula (II): 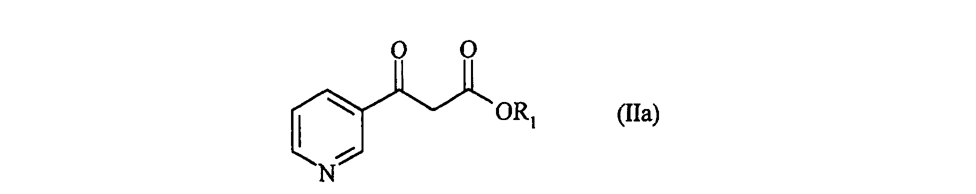 wherein R₁ has the same meaning as hereinbefore,
is reacted with the compound of formula (IIIa), a particular case of the compounds of formula (III): 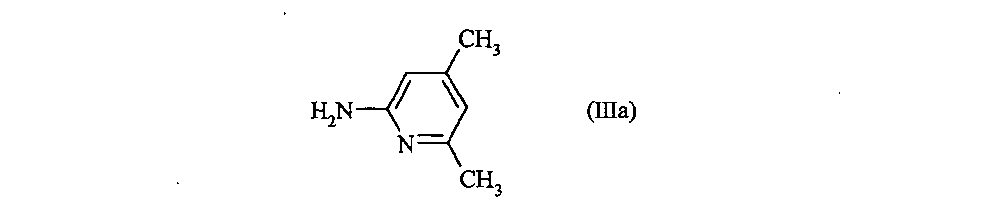 to yield the compound of formula (IVa), a particular case of the compounds of formula (IV): 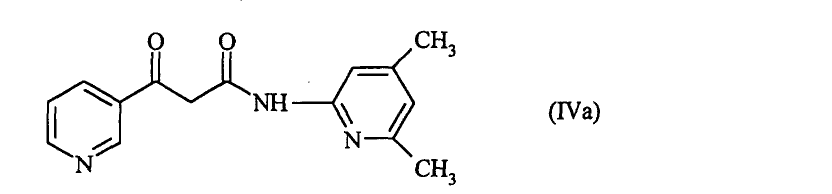 which is reacted with a thionation agent, such as, for example, Lawesson's reagent, to yield 3-(3-pyridyl)-5-[N-(4,6-dimethyl-2-pyridyl)-imino]-1,2-dithiol,
which is purified according to a conventional purification technique, and which is converted, if desired, into addition salts with a pharmaceutically acceptable acid.

7. Synthesis intermediate of 3-(3-pyridyl)-5-[N-(4,6-dimethyl-2-pyridyl)-imino]-1,2-dithiol, which is N-(4,6-dimethyl-2-pyridyl)-3-oxo-3-(3-pyridyl)-propionamide.

8. Pharmaceutical compositions comprising as active ingredient a compound according to any one of claims 1 to 4, alone or in combination with one or more pharmaceutically acceptable, inert, non-toxic carriers.

9. Pharmaceutical compositions according to claim 8, for use in the manufacture of antiinflammatory medicaments.

10. Pharmaceutical compositions according to claim 9, for use in the manufacture of medicaments useful in the treatment of psoriasis.
